# EUROPEAN PATENT SPECIFICATION

(11) **EP 3 921 563 B1**
(45) Date of publication and mention of the grant of the patent: **09.08.2023**
(21) Application number: 19842729.6
(22) Date of filing: 27.12.2019
(51) Int. Cl.: F16L 11/12, F16L 11/24, F16L 47/02, A61M 39/12, F16L 33/34

(54) **INFUSION SET ASSEMBLY**
INFUSIONSET
ASSEMBLAGE POUR INFUSION

(30) Priority: 04.02.2019 US 201916266877
(43) Date of publication of application: 15.12.2021
(73) Proprietor: CareFusion 303, Inc., San Diego, CA 92130 (US)
(72) Inventor: SUWITO, Wantjinarjo, Irvine, California 92604 (US); JEDRZEJEWSKI, Christopher Kazimierz, Alpine, California 91901 (US); WANG, Aaron En-Yu, Laguna Hills, California 92653 (US); CHRISTENSEN, Corey Mark, Anaheim, California 92808 (US)
(74) Representative: Epping - Hermann - Fischer
(86) International application number: PCT/US2019/068810
(87) International publication number: WO 2020/163024

(56) References cited:
- EP-A1- 3 379 126
- US-A- 2 449 265
- US-A1- 2017 321 825

## Description

### CROSS-REFERENCES TO RELATED APPLICATIONS

This application claims the benefit of U.S. Patent Application No. 16/266,877, filed February 4, 2019.

### BACKGROUND

Infusion sets are constructed by joining multiple translucent polymeric tubing segments to multiple polymeric components that are either translucent or opaque. The joints are typically formed by applying a thin layer of solvent or adhesive on one or both of the contacting surfaces and then the two surfaces are brought together. The contacting surface of the tubing can either be at the inner diameter, the outer diameter, or both diameters up to a certain length from one end. The solvent/adhesive is applied either internally, externally, or both. The bonded area, or the bond length for a given diameter, is a critical parameter that if not controlled can cause the infusion sets to leak or separate easily. The bond parameter can vary significantly due to design shortfalls, assembly process shortfalls and process drifts.

Inspecting this critical bond length can be done by visual inspection of the solvent/adhesive wetted area along the contacting surfaces. Another method is inspecting the gap between the tubing end and a hard stop within the component. Both require inspections through translucent components. However, if the component is wavy, textured or opaque, then a visual inspection is difficult to perform, or it cannot be done at all. It is desirable to provide for reliable visual inspections for all infusion set construction regardless of translucency of the components to minimize leaks, separations and variations in total infusion set length.

US20170321825A1 is directed to tubing including insertion reference markings on its outer surface that provide an indicator of how far the tubing is inserted into a fitting. In one embodiment, there is a first insertion reference marking and a second insertion reference marking. A distance between the first insertion reference marking and the second insertion reference marking is a predetermined distance. The first insertion reference marking and the second insertion reference marking provide an indicator of how far the tubing is inserted into a fitting.
EP3379126A1 relates to a connection device (100) for connecting fluid-carrying lines (300) to a line connection (101) which is formed on an end face (102) of the connection device (100), wherein the line connection (101) has a wall (107 ), wherein the wall (107) has a inner wall side (121) which limits a receiving area (111) which is adapted to receive a fluid-carrying line (300), and wherein the wall (107) has an outer wall side (123); and at least one fixing portion (125-1, 125-2, 131) formed on the outer wall side (123) and connectable with at least one fixing member for fixing the connecting device (100).

### SUMMARY

The present disclosure provides tube markers for tubing used in infusion sets to provide an external visual indicator of the fit between an infusion tube and an infusion set component.
The invention is defined by the claims. In the following, parts of the description and drawings referring to embodiments which are not covered by the claims are not presented as embodiments of the invention, but as examples useful for understanding the invention.

According to the invention, an infusion set assembly comprises an infusion component and a tube coupled to the infusion component. The tube comprises a body defining a fluid flow pathway, a first tube end, a second tube end and a tube marker disposed near the first tube end, wherein the tube marker is positioned so that a distance between a leading edge of the tube marker and the outermost surface of the first tube end is substantially equal to a distance between an external edge and a hard stop of a fluid port of the infusion component. The tube comprises a bond area defined between a portion of the outermost surface of the first tube end and an internal surface of the fluid port of the infusion component, the bond area comprising one of an adhesive and a solvent.

In one or more aspects, the first tube end is a fluid input end configured to be inserted into a fluid outlet port of the infusion set component. In one or more aspects, the first tube end is a fluid output end configured to be inserted into a fluid inlet port of the infusion set component. In one or more aspects, the tube marker includes one or more cylindrical bands. In one or more aspects, the tube marker is pad printed ink, laser etching or a tube sleeve. In one or more aspects, the tube sleeve includes a tapered insertion portion configured to fit inside a portion of the fluid port. In one or more aspects, the tapered insertion portion is configured to displace excess solvent. In one or more aspects, the tube sleeve is configured as a stress relief transition from a stiffness of the infusion set component to a lesser stiffness of the tube.

In one or more aspects, the tube includes a second tube marker disposed near the second tube end, wherein the second tube marker is positioned so that a distance between a leading edge of the second tube marker and the outermost surface of the second tube end is substantially equal to a distance between an outermost surface of a second infusion set component fluid port and a hard stop of the second infusion set component fluid port. In one or more aspects, the first tube end is a fluid input end configured to be inserted into a fluid outlet port of the first infusion set component and the second tube end is a fluid output end configured to be inserted into a fluid inlet port of the second infusion set component. In one or more aspects, the first tube end is a fluid output end configured to be inserted into a fluid inlet port of the first infusion set component and the second tube end is a fluid input end configured to be inserted into a fluid outlet port of the second infusion set component.

In one or more aspects, the tube marker includes one of pad printed ink, laser etching and a tube sleeve. In one or more aspects, the tube sleeve includes a tapered insertion portion configured to fit inside a portion of the fluid port, the tapered insertion portion configured to displace a portion of a volume of a bond pocket formed between the tube and the fluid port and a stop portion configured to prevent insertion of the stop portion inside the fluid port. In one or more aspects, the first tube end is one of a fluid input end inserted into a fluid outlet port of the infusion component and a fluid output end inserted into a fluid inlet port of the infusion component. In one or more aspects, the tube marker includes at least one cylindrical band. In one or more aspects, the infusion set assembly includes a second infusion component and a second tube marker disposed near the second tube end. The second tube marker is positioned so that a distance between a leading edge of the second tube marker and the outermost surface of the second tube end is substantially equal to a distance between an external edge and a hard stop of a fluid port of the second infusion component.

According to the invention, a method of manufacturing the infusion set assembly as described above is disclosed. The method comprises:
providing an infusion component having a fluid port;
disposing a tube marker near a first tube end of a tube, the tube comprising a body defining a fluid flow pathway, the first tube end and a second tube end;
positioning the tube marker so that a distance between a leading edge of the tube marker and an outermost surface of the first tube end is substantially equal to a distance between an external edge and a hard stop of the fluid port of the infusion component;
applying an adhesive or a solvent to one of the first tube end and the fluid port of the infusion component; and
coupling the first tube end to the fluid port of the infusion component so that a bond area of the adhesive or the solvent is defined between a portion of the outermost surface of the first tube end and an internal surface of the fluid port of the infusion component.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 depicts a schematic view of a typical assembled infusion set.
FIG. 2 depicts a schematic view of a typical infusion set component and tubing prior to assembly.
FIG. 3 depicts a cross-section view of the infusion set component of FIG. 2.
FIG. 4 depicts a schematic view of the infusion set component and tubing of FIG. 2 after assembly.
FIG. 5 depicts a cross-section view of the infusion set component and tubing of FIG. 4.
FIG. 6 depicts a schematic view of one or more embodiments of the invention of an assembled infusion set component and tubing with markers.
FIG. 7 depicts a cross-sectional view of the infusion set component of FIG. 6.
FIG. 8 depicts a schematic view of one or more embodiments of the invention of a tubing segment with markers.
FIG. 9 depicts a cross-sectional view of a typical infusion set component joined with a tubing segment.
FIG. 10 depicts a schematic view of one or more embodiments of the invention of a tubing segment with a sleeve.
FIG. 11 depicts a cross-sectional view of one or more embodiments of the invention of an infusion set component joined with the tubing segment of FIG. 10.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Infusion sets may be formed from any combination of infusion components and tubing. Typically, the infusion components and tubing are disposable products that are used once and then discarded. The infusion components and tubing may be formed from any suitable material (e.g., plastic, silicone, rubber). An issue in manufacturing infusion sets is consistently joining the tubing and the infusion components to obtain a secure and/or leak free joint with desired fluid flow. The joint may be formed at either the inner or outer diameter of the tubing. In some cases, the joint may be formed both by bonding inner and outer diameters of the tubing to a component for additional joint strength.

Prior approaches provide a partial solution by making the infusion components translucent. However, even inspecting through a translucent body can be challenging when the outer surface is not smooth or additional layers of material are present, such as a textured gripping surface. In some cases, the infusion components are made opaque and with different colors to indicate a fluid flow direction (e.g., a check valve). Since seeing through opaque material is difficult to impossible, another prior approach is that the joint is typically made at the inner diameter of the tubing. This restricts fluid flow due to a smaller orifice than the tubing inner diameter at the joint. Additionally, applying solvent/adhesive on the inside of the tubing has a higher propensity for occlusions, because the solvent/adhesive is pushed in as the tube is inserted to form a joint.

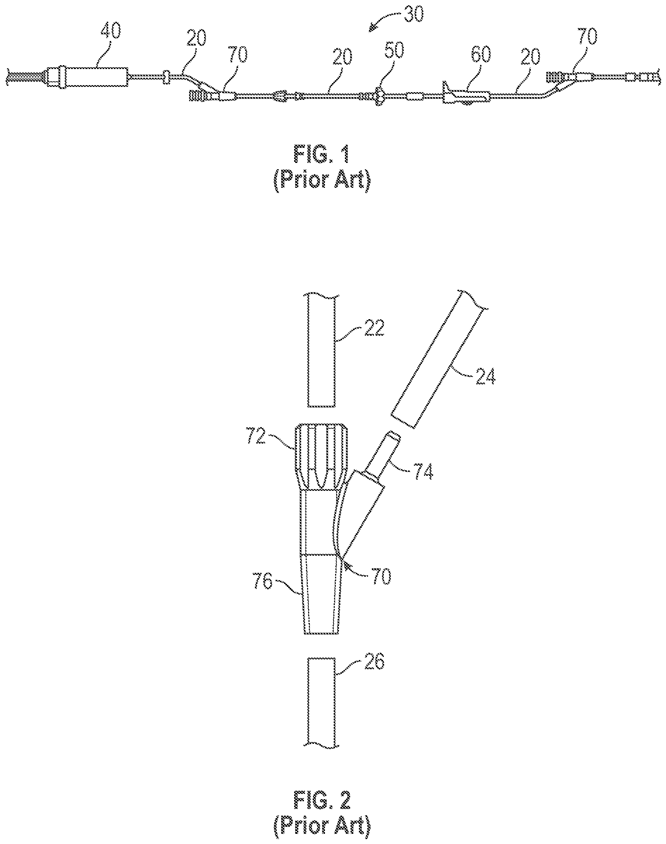

As shown in FIG. 1, a typical infusion set 30 may include a drip chamber 40, a check valve 50, a roller clamp 60 and Y-junctions 70, all connected together by tubing 20. A typical infusion set 30 can include additional infusion components and can be formed of any combination of components and the tubing 20.

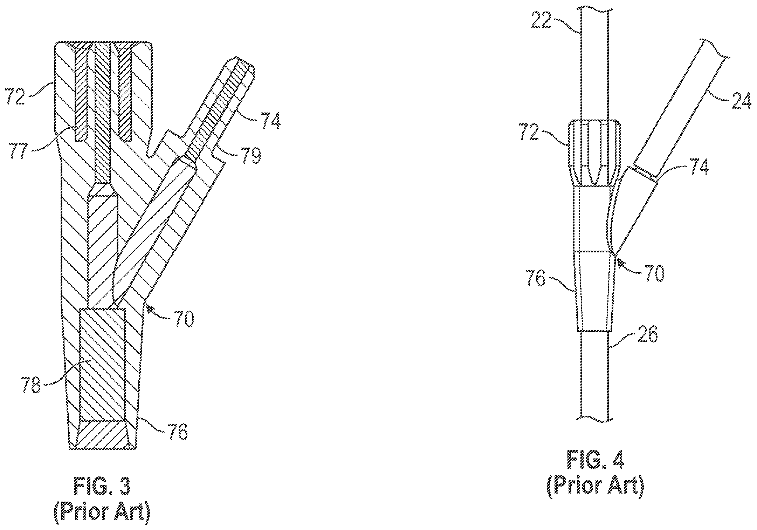

As shown in FIG. 2, a typical Y-junction 70 has inlet ports 72, 74 and an outlet port 76, where each inlet port 72, 74 is to be connected to an inlet tube 22, 24 and the outlet port 76 is to be connected to an outlet tube 26. As shown in FIG. 3, the inlet port 72 has an internal joint 77 where the inlet tube 22 is to be inserted into the inlet port 72. Similarly, the outlet port 76 has an internal joint 78 where the outlet tube 26 is to be inserted into the outlet port 76. By contrast, the inlet port 74 has an external joint 79 where the inlet tube 24 is to be slid onto and over the inlet port 74.

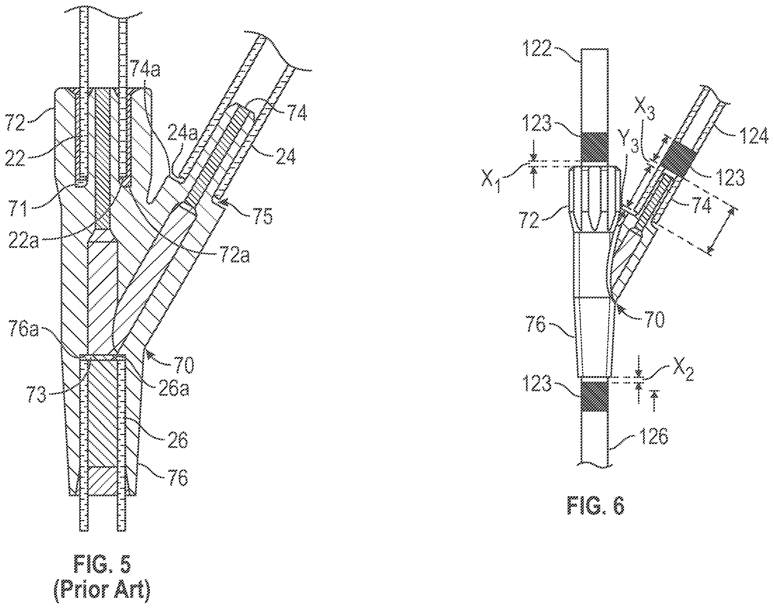

FIGS. 4 and 5 show the Y-junction 70 and the inlet/outlet tubes 22, 24, 26 after assembly. An incomplete joining of the inlet tube 22 with the inlet port 72 leaves a gap 71 between an end surface 22a of the inlet tube 22 and an engagement surface 72a of the inlet port 72. Similarly, an incomplete joining of the outlet tube 26 with the outlet port 76 leaves a gap 73 between an end surface 26a of the outlet tube 26 and an engagement surface 76a of the outlet port 76. Further, an incomplete joining of the inlet tube 24 with the inlet port 74 leaves a gap 75 between an end surface 24a of the inlet tube 24 and an engagement surface 74a of the inlet port 74. As discussed above, the gaps 71, 73, 75 increase the likelihood that the respective internal/external joints 77, 78, 79 will fail, either by leaking or by separation of the inlet/outlet tubes 22, 24, 26 from the Y-junction 70.

Unobstructed visual inspections on sharp edges with good contrast lend themselves for good repeatability and reproducibility (R&R). This is more so for automated computer vision inspections. An acceptable gage R&R is required for measuring critical parameters, such as the case for bond joint area/length. This critical parameter is currently measured by measuring the gap between the tubing end and the insertion hard stop within the component. This method has been found difficult to do in some cases and impossible in others.

By adding sharp contrasting band markers along the tubing, a necessary element to make a good distance measurement is provided. The infusion components are typically injection molded with great dimensional precision. The distance between the hard stop and an external edge can generally be deduced from a drawing. Therefore, the distance between the band marker and that external edge can be used to deduce the bond length.

The marker bands or markers may be produced by a pad printing method, laser marking, or any other suitable method, as long as good contrasting markers are produced. The markers may be high contrasting solid bands. The bands may be two or more fragmented blobs at the outer diameter of tubing. The shape may be a band or any other suitable shape that can be easily seen and analyzed with unaided eyes or computer vision, as well as does not block fluid flow inspection. The addition of markers and others on a translucent tubing may be 40% or less on every inch of the tubing surface area.

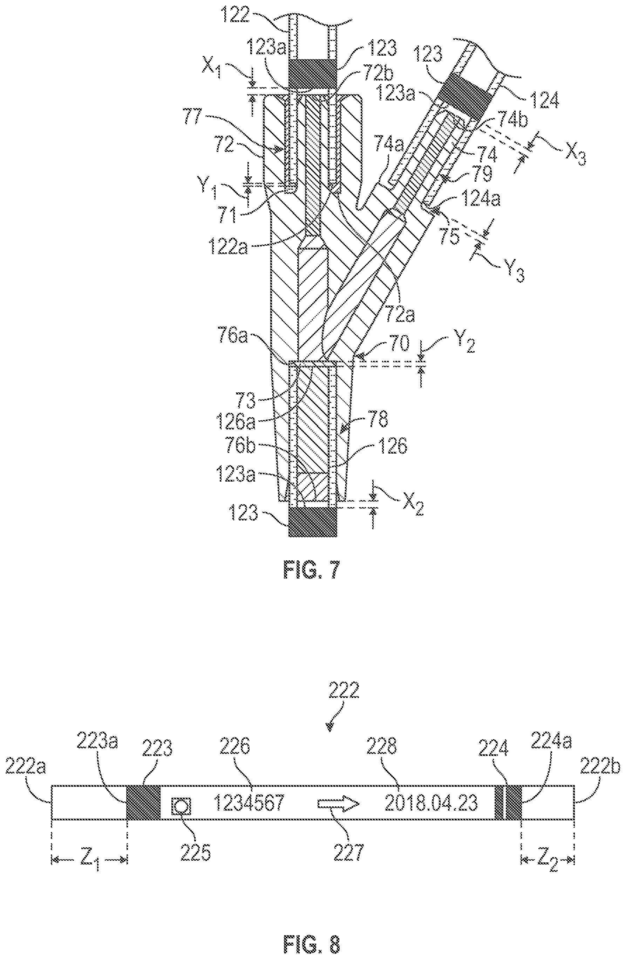

As shown in FIGS. 6 and 7, a Y-joint 70 may be joined together with inlet tubes 122, 124 and outlet tube 126. The inlet tube 122 includes a tube marker 123, which is disposed at a specific position on the inlet tube 122. Thus, when a leading edge 123a of the tube mark 123 is aligned with (e.g., even with) the top surface 72b of the inlet port 122, there is no gap between the end surface 122a of the inlet tube 122 and the engagement surface 72a of the inlet port 72 in the internal joint 77. As another example, if there is a gap 71 between the end surface 122a of the inlet tube 122 and the engagement surface 72a of the inlet port 72, the distance x₁ between the leading edge 123a of the tube mark 123 and the top surface 72b of the inlet port 72 is commensurate or substantially equal to the distance y₁ of gap 71.

Similarly, for the internal joint 78 formed by the outlet tube 126 and the outlet port 76, the outlet tube 126 includes the tube marker 123 disposed at a specific position on the outlet tube 126. Here, when a leading edge 123a of the tube mark 123 is aligned with (e.g., even with) the bottom surface 76b of the outlet port 76, there is no gap between the end surface 126a of the outlet tube 126 and the engagement surface 76a of the outlet port 76. If there is a gap 73 between the end surface 126a of the outlet tube 126 and the engagement surface 76a of the outlet port 76, the distance x₂ between the leading edge 123a of the tube mark 123 and the bottom surface 76b of the outlet port 76 is commensurate or substantially equal to the distance y₂ of gap 73.

For the external joint 79 formed by the inlet tube 124 and the inlet port 74, the inlet tube 124 includes the tube marker 123 disposed at a specific position on the inlet tube 124. When a leading edge 123a of the tube mark 123 is aligned with (e.g., even with) the top surface 74b of the outlet port 74, there is no gap between the end surface 124a of the inlet tube 124 and the engagement surface 74a of the inlet port 74. If there is a gap 75 between the end surface 124a of the inlet tube 124 and the engagement surface 74a of the inlet port 74, the distance x₃ between the leading edge 123a of the tube mark 123 and the top surface 74b of the inlet port 74 is commensurate or substantially equal to the distance y₃ of gap 75.

As shown in FIG. 8, a tube 222 may include markers 223, 224 at both ends for providing visual guidance during joining of the tube 222 to infusion set components. The markers 223, 224 may have different characteristics or looks based on different marker distances from the tube ends. For example, the marker 223 may be a single solid band that is associated with a distance zi between the leading edge 223a of the marker 223 and the first tube end 222a. As another example, the marker 224 may be two different sized solid bands that are associated with a distance z₂ between the leading edge 224a of the marker 224 and the second tube end 222b. The tube 222 may also include one or more informational markings, such as a logo 225, a part number/unique device identification 226, a symbol 227 and a date code 228. The informational markings may be disposed between the markers 223, 224 so that the portions of the tube 222 from the leading edge of the markers 223a, 224a to the tube ends 222a, 222b remain clear of markings for ease of use of markers 223, 224. The informational markings may provide relevant information regarding the tubing 222, the infusion set components and/or the infusion set. Thus, the relevant information may be easily discerned from the tubing 222 instead of having to be determined from the associated packaging that is typically discarded.

Another related aspect of the disclosure is determining when and where to add the markers. Infusion set tubing is typically produced using an extrusion method and spooled. The spooled tubing is then cut to desired lengths and the cut tubing is then joined to infusion set components to make an infusion set. The present disclosure provides for a marking tool (e.g., pad printer, laser) to be disposed in a cutting station in which the tubing may be cut to a precise length and markers may be applied at the same cutting and marking machine/station. Thus, no additional process time is incurred by moving materials, loading materials and adding the markers on a different machine/station.

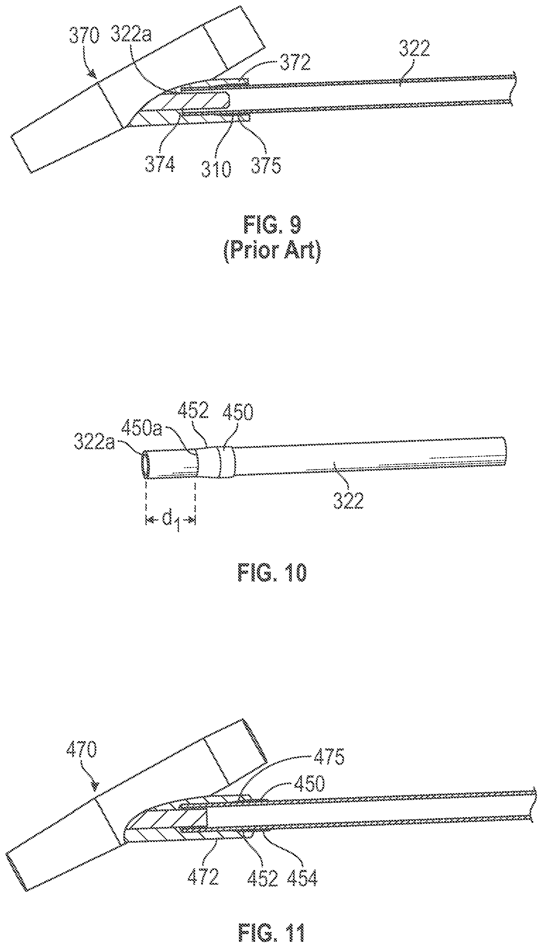

FIG. 9 shows a Y-joint 370 having an inlet port 372 joined or bonded to a typical tube 322 using solvent/adhesive. The inlet port 372 includes a stop member 374 that provides a hard stop when the tube end 322a butts up against the stop member 374 during insertion of the tube 322 into the inlet port 372. A solvent/adhesive pool 310 may form inside a lead-in chamfer of a bond pocket 375. The solvent/adhesive pool 310 may degrade the tube 322 as the solvent/adhesive is curing.

As shown in FIGS. 10 and 11, a tube sleeve 450 may be added onto the tube 322 so that the leading edge 450a of the tube sleeve 450 is a specific distance di from the tube end 322a. The tube sleeve 450 may have a taper such that an insertion portion 452 is sized and shaped to fit inside a part of an infusion set component, such as an inlet port 472 of a Y-junction 470. A stop portion 454 of the tube sleeve 450 may be sized and shaped not to fit into the infusion set component (e.g., inlet port 472), thus preventing a portion of the tube sleeve from sliding into the inlet port 472 in this example. The tube sleeve 450 may eliminate the need to have a mechanical stop inside the infusion set component (e.g., stop member 374 of Y-junction 370), while ensuring that the tube 322 is inserted the correct distance into the inlet port 472.

The tube sleeve 450 may be connected to tube 322 in any suitable manner, such as over molding or heat shrinking into place. The tube sleeve 450 may provide a stress relief transition from a stiff plastic component (e.g., Y-junction 470) to a softer tubing (e.g., tube 322). The tube sleeve 450 may also provide kink resistance to the tube 322 when the tube is confined within packaging. The insertion portion 452 the tube sleeve 450 may further provide for displacement of a portion of the volume of the bond pocket 475, thus preventing the pooling of excess solvent/adhesive.

## Claims

1. An infusion set assembly (30), comprising:
an infusion component; and
a tube (122, 124, 126, 222, 232) coupled to the infusion component, the tube (122, 124, 126, 222, 232) comprising:
a body defining a fluid flow pathway;
a first tube end (222a);
a second tube end (222b); and
a tube marker (223, 224) disposed near the first tube end (222a), wherein the tube marker (223, 224) is positioned so that a distance between a leading edge (223a, 224a, 450a) of the tube marker (223, 224) and the outermost surface of the first tube end (222a) is substantially equal to a distance between an external edge and a hard stop of a fluid port of the infusion component; and
a bond area defined between a portion of the outermost surface of the first tube end (222a) and an internal surface of the fluid port of the infusion component, the bond area comprising one of an adhesive and a solvent.

2. The infusion set assembly (30) of Claim 1, wherein the tube marker (223, 224) comprises a cylindrical band.

3. The infusion set assembly (30) of Claim 1, wherein the tube marker (223, 224) comprises multiple cylindrical bands.

4. The infusion set assembly (30) of Claim 1, wherein the tube marker (223, 224) comprises pad printed ink.

5. The infusion set assembly (30) of Claim 1, wherein the tube marker (223, 224) comprises laser etching.

6. The infusion set assembly (30) of Claim 1, wherein the tube marker (223, 224) comprises a tube sleeve (450).

7. The infusion set assembly (30) of Claim 6, wherein the tube sleeve (450) comprises:
a tapered insertion portion configured to fit inside a portion of the fluid port.

8. The infusion set assembly (30) of Claim 7, wherein the tapered insertion portion is configured to displace excess solvent.

9. The infusion set assembly (30) of Claim 6, wherein the tube sleeve (450) is configured as a stress relief transition from a stiffness of the infusion set component to a lesser stiffness of the tube (122, 124, 126, 222, 232).

10. The infusion set assembly (30) of Claim 1, further comprising a second tube marker (223, 224) disposed near the second tube end (222b), wherein the second tube marker (223, 224) is positioned so that a distance between a leading edge (223a, 224a, 450a) of the second tube marker (223, 224) and the outermost surface of the second tube end (222b) is substantially equal to a distance between an outermost surface of a second infusion set component fluid port and a hard stop of the second infusion set component fluid port.

11. The infusion set assembly (30) of Claim 10, wherein the first tube end (222a) is a fluid input end configured to be inserted into a fluid outlet port of the first infusion set component and the second tube end (222b) is a fluid output end configured to be inserted into a fluid inlet port of the second infusion set component.

12. The infusion set assembly (30) of Claim 10, wherein the first tube end (222a) is a fluid output end configured to be inserted into a fluid inlet port of the first infusion set component and the second tube end (222b) is a fluid input end configured to be inserted into a fluid outlet port of the second infusion set component.

13. A method of manufacturing the infusion set assembly (30) of anyone of claims 1 to 12, the method comprising:
providing an infusion component having a fluid port;
disposing a tube marker (223, 224) near a first tube end of a tube (122, 124, 126, 222, 232), the tube (122, 124, 126, 222, 232) comprising a body defining a fluid flow pathway, the first tube end and a second tube end;
positioning the tube marker (223, 224) so that a distance between a leading edge (223a, 224a, 450a) of the tube marker (223, 224) and an outermost surface of the first tube end (222a) is substantially equal to a distance between an external edge and a hard stop of the fluid port of the infusion component;
applying an adhesive or a solvent to one of the first tube end (222a) and the fluid port of the infusion component; and
coupling the first tube end (222a) to the fluid port of the infusion component so that a bond area of the adhesive or the solvent is defined between a portion of the outermost surface of the first tube end (222a) and an internal surface of the fluid port of the infusion component.

14. The method of claim 13, wherein disposing the tube marker (223, 224) near the first tube end (222a) of the tube (122, 124, 126, 222, 232) comprises one of:
printing the tube marker (223, 224) with pad printed ink;
etching the tube marker (223, 224) with a laser; and
coupling a tube sleeve (450) to the tube (122, 124, 126, 222, 232).

15. The method of claim 14, wherein if the tube marker (223, 224) is a tube sleeve (450), further comprising:
inserting a tapered portion of the tube sleeve (450) into a portion of the fluid port of the infusion component; and
displacing excess adhesive or solvent with the tapered portion of the tube sleeve (450).

## Patentansprüche

1. Infusionsset-Baugruppe (30), aufweisend:
ein Infusionsbauteil; und
einen Schlauch (122, 124, 126, 222, 232), der mit dem Infusionsbauteil verbunden ist, wobei der Schlauch (122, 124, 126, 222, 232) aufweist:
einen Hauptteil, der einen Fluidströmungsweg bildet;
ein erstes Schlauchende (222a);
ein zweites Schlauchende (222b); und
eine Schlauchmarkierung (223, 224), die nahe dem ersten Schlauchende (222a) angeordnet ist, wobei die Schlauchmarkierung (223, 224) so positioniert ist, dass ein Abstand zwischen einer Vorderkante (223a, 224a, 450a) der Schlauchmarkierung (223, 224) und der äußersten Oberfläche des ersten Schlauchendes (222a) im Wesentlichen gleich einem Abstand zwischen einem äußeren Rand und einem harten Anschlag eines Fluidanschlusses des Infusionsbauteils ist; und
eine Klebefläche, die zwischen einem Abschnitt der äußersten Oberfläche des ersten Schlauchendes (222a) und einer inneren Oberfläche des Fluidanschlusses des Infusionsbauteils gebildet ist, wobei die Klebefläche ein Klebemittel oder ein Lösungsmittel aufweist.

2. Infusionsset-Baugruppe (30) nach Anspruch 1, wobei die Schlauchmarkierung (223, 224) ein zylindrisches Band aufweist.

3. Infusionsset-Baugruppe (30) nach Anspruch 1, wobei die Schlauchmarkierung (223, 224) mehrere zylindrische Bänder aufweist.

4. Infusionsset-Baugruppe (30) nach Anspruch 1, wobei die Schlauchmarkierung (223, 224) eine über Tampondruck aufgebrachte Druckfarbe aufweist.

5. Infusionsset-Baugruppe (30) nach Anspruch 1, wobei die Schlauchmarkierung (223, 224) eine Lasergravierung aufweist.

6. Infusionsset-Baugruppe (30) nach Anspruch 1, wobei die Schlauchmarkierung (223, 224) eine Schlauchhülse (450) aufweist.

7. Infusionsset-Baugruppe (30) nach Anspruch 6, wobei die Schlauchhülse (450) aufweist:
einen sich verjüngenden Einführabschnitt, der so ausgelegt ist, dass er in einen Abschnitt des Fluidanschlusses hineinpasst.

8. Infusionsset-Baugruppe (30) nach Anspruch 7, wobei der sich verjüngende Einführabschnitt so ausgelegt ist, dass er überschüssiges Lösungsmittel verdrängt.

9. Infusionsset-Baugruppe (30) nach Anspruch 6, wobei die Schlauchhülse (450) als Spannungsabbauübergang von einer Steifigkeit des Infusionsset-Bauteils zu einer geringeren Steifigkeit des Schlauchs (122, 124, 126, 222, 232) ausgelegt ist.

10. Infusionsset-Baugruppe (30) nach Anspruch 1, darüber hinaus mit einer zweiten Schlauchmarkierung (223, 224), die nahe dem zweiten Schlauchende (222b) angeordnet ist, wobei die zweite Schlauchmarkierung (223, 224) so positioniert ist, dass ein Abstand zwischen einer Vorderkante (223a, 224a, 450a) der zweiten Schlauchmarkierung (223, 224) und der äußersten Oberfläche des zweiten Schlauchendes (222b) im Wesentlichen gleich einem Abstand zwischen einer äußersten Oberfläche eines zweiten Infusionsset-Bauteil-Fluidanschlusses und einem harten Anschlag des zweiten Infusionsset-Bauteil-Fluidanschlusses ist.

11. Infusionsset-Baugruppe (30) nach Anspruch 10, wobei das erste Schlauchende (222a) ein Fluideingangsende ist, das dafür ausgelegt ist, in einen Fluidauslassanschluss des ersten Infusionsset-Bauteils eingeführt zu werden, und das zweite Schlauchende (222b) ein Fluidausgangsende ist, das dafür ausgelegt ist, in einen Fluideinlassanschluss des zweiten Infusionsset-Bauteils eingeführt zu werden.

12. Infusionsset-Baugruppe (30) nach Anspruch 10, wobei das erste Schlauchende (222a) ein Fluidausgangsende ist, das dafür ausgelegt ist, in einen Fluideinlassanschluss des ersten Infusionsset-Bauteils eingeführt zu werden, und das zweite Schlauchende (222b) ein Fluideingangsende ist, das dafür ausgelegt ist, in einen Fluidauslassanschluss des zweiten Infusionsset-Bauteils eingeführt zu werden.

13. Verfahren zur Herstellung der Infusionsset-Baugruppe (30) nach einem der Ansprüche 1 bis 12, wobei das Verfahren umfasst:
Bereitstellen eines Infusionsbauteils mit einem Fluidanschluss;
Anordnen einer Schlauchmarkierung (223, 224) nahe einem ersten Schlauchende eines Schlauchs (122, 124, 126, 222, 232), wobei der Schlauch (122, 124, 126, 222, 232) einen Hauptteil, der einen Fluidströmungsweg bildet, das erste Schlauchende und ein zweites Schlauchende aufweist;
Positionieren der Schlauchmarkierung (223, 224) in der Weise, dass ein Abstand zwischen einer Vorderkante (223a, 224a, 450a) der Schlauchmarkierung (223, 224) und einer äußersten Oberfläche des ersten Schlauchendes (222a) im Wesentlichen gleich einem Abstand zwischen einem äußeren Rand und einem harten Anschlag des Fluidanschlusses des Infusionsbauteils ist;
Auftragen eines Klebemittels oder eines Lösungsmittels auf das erste Schlauchende (222a) oder den Fluidanschluss des Infusionsbauteils; und
Verbinden des ersten Schlauchendes (222a) mit dem Fluidanschluss des Infusionsbauteils in der Weise, dass eine Klebefläche des Klebemittels oder des Lösungsmittels zwischen einem Abschnitt der äußersten Oberfläche des ersten Schlauchendes (222a) und einer inneren Oberfläche des Fluidanschlusses des Infusionsbauteils gebildet wird.

14. Verfahren nach Anspruch 13, wobei das Anordnen der Schlauchmarkierung (223, 224) nahe dem ersten Schlauchende (222a) des Schlauchs (122, 124, 126, 222, 232) einen der folgenden Vorgänge umfasst:
Drucken der Schlauchmarkierung (223, 224) mit einer über Tampondruck aufgebrachten Druckfarbe;
Gravieren der Schlauchmarkierung (223, 224) mit einem Laser; und
Verbinden einer Schlauchhülse (450) mit dem Schlauch (122, 124, 126, 222, 232).

15. Verfahren nach Anspruch 14, wobei, wenn die Schlauchmarkierung (223, 224) eine Schlauchhülse (450) ist, es darüber hinaus umfasst:
Einführen eines sich verjüngenden Abschnitts der Schlauchhülse (450) in einen Abschnitt des Fluidanschlusses des Infusionsbauteils; und
Verdrängen von überschüssigem Klebemittel oder Lösungsmittel mit dem sich verjüngenden Abschnitt der Schlauchhülse (450).

## Revendications

1. Ensemble kit de perfusion (30), comprenant :
un composant de perfusion ; et
un tube (122, 124, 126, 222, 232) couplé au composant de perfusion, le tube (122, 124, 126, 222, 232) comprenant :
un corps définissant une voie d'écoulement de fluide ;
une première extrémité de tube (222a) ;
une deuxième extrémité de tube (222b) ; et
un marqueur de tube (223, 224) disposé près de la première extrémité de tube (222a), sachant que le marqueur de tube (223, 224) est positionné de telle sorte qu'une distance entre un bord d'attaque (223a, 224a, 450a) du marqueur de tube (223, 224) et la surface la plus extérieure de la première extrémité de tube (222a) soit sensiblement égale à une distance entre un bord externe et une butée dure d'un orifice de fluide du composant de perfusion ; et
une zone de liaison définie entre une partie de la surface la plus extérieure de la première extrémité de tube (222a) et une surface interne de l'orifice de fluide du composant de perfusion, la zone de liaison comprenant l'un d'un adhésif et d'un solvant.

2. L'ensemble kit de perfusion (30) de la revendication 1, sachant que le marqueur de tube (223, 224) comprend une bande cylindrique.

3. L'ensemble kit de perfusion (30) de la revendication 1, sachant que le marqueur de tube (223, 224) comprend de multiples bandes cylindriques.

4. L'ensemble kit de perfusion (30) de la revendication 1, sachant que le marqueur de tube (223, 224) comprend de l'encre imprimée au tampon.

5. L'ensemble kit de perfusion (30) de la revendication 1, sachant que le marqueur de tube (223, 224) comprend une gravure au laser.

6. L'ensemble kit de perfusion (30) de la revendication 1, sachant que le marqueur de tube (223, 224) comprend un manchon de tube (450).

7. L'ensemble kit de perfusion (30) de la revendication 6, sachant que le manchon de tube (450) comprend :
une partie d'insertion conique configurée pour s'ajuster à l'intérieur d'une partie de l'orifice de fluide.

8. L'ensemble kit de perfusion (30) de la revendication 7, sachant que la partie d'insertion conique est configurée pour évacuer du solvant excédentaire.

9. L'ensemble kit de perfusion (30) de la revendication 6, sachant que le manchon de tube (450) est configuré comme une transition de détente de contrainte depuis une rigidité du composant de kit de perfusion à une rigidité moindre du tube (122, 124, 126, 222, 232).

10. L'ensemble kit de perfusion (30) de la revendication 1, comprenant en outre un deuxième marqueur de tube (223, 224) disposé près de la deuxième extrémité de tube (222b), sachant que le deuxième marqueur de tube (223, 224) est positionné de telle sorte qu'une distance entre un bord d'attaque (223a, 224a, 450a) du deuxième marqueur de tube (223, 224) et la surface la plus extérieure de la deuxième extrémité de tube (222b) soit sensiblement égale à une distance entre une surface la plus extérieure d'un deuxième orifice de fluide de composant de kit de perfusion et une butée dure du deuxième orifice de fluide de composant de kit de perfusion.

11. L'ensemble kit de perfusion (30) de la revendication 10, sachant que la première extrémité de tube (222a) est une extrémité d'entrée de fluide configurée pour être insérée dans un orifice de sortie de fluide du premier composant kit de perfusion et la deuxième extrémité de tube (222b) est une extrémité de sortie de fluide configurée pour être insérée dans un orifice d'entrée de fluide du deuxième composant de kit de perfusion.

12. L'ensemble kit de perfusion (30) de la revendication 10, sachant que la première extrémité de tube (222a) est une extrémité de sortie de fluide configurée pour être insérée dans un orifice d'entrée de fluide du premier composant de kit de perfusion et la deuxième extrémité de tube (222b) est une extrémité d'entrée de fluide configurée pour être insérée dans un orifice de sortie de fluide du deuxième composant de kit de perfusion.

13. Procédé de fabrication de l'ensemble kit de perfusion (30) de l'une quelconque des revendications 1 à 12, le procédé comprenant :
la fourniture d'un composant de perfusion présentant un orifice de fluide ;
la disposition d'un marqueur de tube (223, 224) près d'une première extrémité de tube d'un tube (122, 124, 126, 222, 232), le tube (122, 124, 126, 222, 232) comprenant un corps définissant une voie d'écoulement de fluide, la première extrémité de tube et une deuxième extrémité de tube ;
le positionnement du marqueur de tube (223, 224) de telle sorte qu'une distance entre un bord d'attaque (223a, 224a, 450a) du marqueur de tube (223, 224) et une surface la plus extérieure de la première extrémité de tube (222a) soit sensiblement égale à une distance entre un bord externe et une butée dure de l'orifice de fluide du composant de perfusion ; et
l'application d'un adhésif ou d'un solvant à l'un de la première extrémité de tube (222a) et de l'orifice de fluide du composant de perfusion ; et
le couplage de la première extrémité de tube (222a) à l'orifice de fluide du composant de perfusion de telle sorte qu'une zone de liaison de l'adhésif ou du solvant soit définie entre une partie de la surface la plus extérieure de la première extrémité de tube (222a) et une surface interne de l'orifice de fluide du composant de perfusion.

14. Le procédé de la revendication 13, sachant que la disposition du marqueur de tube (223, 224) près de la première extrémité de tube (222a) du tube (122, 124, 126, 222, 232) comprend l'un de :
l'impression du marqueur de tube (223, 224) avec de l'encre imprimée au tampon ;
la gravure du marqueur de tube (223, 224) avec un laser ; et
le couplage d'un manchon de tube (450) au tube (122, 124, 126, 222, 232).

15. Le procédé de la revendication 14, sachant que si le marqueur de tube (223, 224) est un manchon de tube (450), le procédé comprend en outre :
l'insertion d'une partie conique du manchon de tube (450) dans une partie de l'orifice de fluide du composant de perfusion ; et
l'évacuation d'adhésif ou de solvant excédentaire avec la partie conique du manchon de tube (450).
